# EUROPEAN PATENT SPECIFICATION

(11) **EP 2 228 082 B1**
(45) Date of publication and mention of the grant of the patent: **13.06.2018**
(21) Application number: 09001709.6
(22) Date of filing: 06.02.2009
(51) Int. Cl.: A61L 31/10, A61L 31/14, A61L 31/16

(54) **Method for preparing drug-eluting stent having nano-structured pattern**
Verfahren zur Herstellung von wirkstofffreisetzenden Stents mit nanostrukturiertem Muster
Procédé de préparation d'une endoprothèse pour élution de médicaments ayant un modèle nano-structuré

(30) Priority: 29.12.2008 KR 20080135808
(43) Date of publication of application: 15.09.2010
(73) Proprietor: Korea Institute of Science and Technology, Seoul 136-791 (KR)
(72) Inventor: Moon, Myoung-Woon, Seoul 151-050 (KR); Lee, Kwang Ryeol, Seoul 137-948 (KR); Han, Dong Keun, Seoul 139-908 (KR); Park, Kwi Deok, Seoul 139-242 (KR)
(74) Representative: advotec.

(56) References cited:
- EP-A2- 1 832 289
- WO-A2-2006/063157
- WO-A2-2009/036373
- US-A1- 2007 224 235
- US-B1- 7 431 959
- MORI H ET AL: "Increased adhesion of diamond-like carbon-Si coatings and its tribological properties", SURFACE AND COATINGS TECHNOLOGY, ELSEVIER BV, AMSTERDAM, NL, vol. 149, no. 2-3, 15 January 2002 (2002-01-15), pages 225-230, XP002524718, ISSN: 0257-8972, DOI: 10.1016/S0257-8972(01)01449-9

## Description

### FIELD OF THE INVENTION

The present invention relates to a method for preparing a drug-eluting stent using a chemical vapor deposition.

### BACKGROUND OF THE INVENTION

Recently, with the population ageing, the demand for vascular implants such as vascular stents for coronary and peripheral arteries is increasing, and thus the importation of these products is also constantly increasing. However, vascular stents can cause acute occlusion due to thrombus formation after implantation, and the stents themselves act as traumatic factors in vascular walls to induce intimal hyperplasia, thus causing a problem of restenosis. For this reason, functional surface modification technology having a drug-eluting function of delivering therapeutic drugs directly into blood vessels has been required together with surface treatment for inhibiting thrombosis. Thus, Hepacoat Corporation et al. has commercially marketed a stent coated with heparin for inhibiting thrombosis, and Cordis Corporation has commercially marketed Cypher™ as a first drug-eluting stent for preventing vascular restenosis. However, problems in that patients implanted with these stents have died recently occurred, and thus the need to develop a stent having improved drug-eluting performance exists.

Meanwhile, studies on diamond-like carbon (DLC) coatings and the activation thereof are being studied. Such coatings are biocompatible on the surface of a material, such as nitinol (TiNi) or stainless steel that is the main material of a general alloy stent loaded with no drug. Also, these coatings protect vascular walls implanted with stents and, in addition, can prevent thrombosis and restenosis. Particularly, various methods for synthesizing amorphous hard carbon films have been developed, and a coated stent is currently being marketed by Phytis AG. Moreover, US Patent Publication No. 2006/0200231 discloses a method for preparing a drug-eluting stent (DES), in which a carbon-containing material and DLC are used and a metal bar is inserted to improve the porosity.

Also, as technologies for imparting biocompatibility, there have been attempts to improve biocompatibility by surface modification with microstructures or nanostructures. For example, methods of modifying the surfaces of materials using an ion beam or plasma were discovered in the first half of the 1990s and were significantly improved through the latter half of the 1990s. However, in most cases, a glassy metal, an amorphous material such as amorphous silicon (a-Si), or a crystalline material such as silicon (Si) were used as materials exposed to ion beams.

Finkelstein et al. attempted to control the kind and release rate of drugs by forming a deep groove in the surface of a metal stent and inserting into the groove a polymer loaded with 2-3 kinds of drugs in multiple layers (Finkelstein et al., Circulation, 107 (2003) 777-784). Also, Ankur Raval et al. attempted to control a drug-loading function by depositing a biodegradable polymer and a non-biodegradable polymer on the surface of a stent in four layers (Ankur Raval, Trends Biomater. Artif. Organs, Vol. 20(2) (2007) 101-110).

However, there has been no case in which the surface of polymers currently being widely used in biological research, for example, a polymer such as (polylactic-co-glycolic acid PLGA), has been modified using ion beams. Furthermore, a study on the formation of a nanostructured pattern for loading drugs on the polymer surface has not yet been conducted.

US7431959 discloses a method for preparing drug eluting medical devices comprising applying a polymer/drug coating and treating it with ion beam/ion plasma to modify the polymer's permeability.

### SUMMARY OF THE INVENTION

Accordingly, it is an object of the present invention to provide a method for preparing a drug-eluting stent having a nanostructured pattern using a plasma-assisted plasma vapor deposition.

In accordance with the present invention, there is provided a method for preparing a drug-eluting stent, comprising the steps of: (a) forming a first biodegradable polymer layer on the surface of a stent; (b) forming a nanostructured pattern on the surface of the first biodegradable polymer layer by treatment with ion beams or plasma using a plasma-assisted chemical vapor deposition (PACVD); and, (c) forming a second biodegradable polymer layer on the first biodegradable polymer layer having the nanostructured pattern formed thereon, at least one of the first and second biodegradable polymer layers being loaded with identical or different drugs, wherein the ion beam or plasma treatment in step (b) is carried out at a voltage ranging from -100 V to -100 kV, wherein the ion beam or plasma treatment in step (b) is carried out at a power ranging from 1 W to 10 kW, and wherein the ion beam or plasma treatment in step (b) is carried out for a time ranging from 1 second to 2 hours. The inventive method for preparing a stent using a plasma-assisted chemical vapor deposition can improve drug-loading capability and control drug elution rate by modifying the surface of a biodegradable polymer with nanostructures. Thus, the method of the present invention is useful for the preparation of a drug-eluting stent.

### BRIEF DESCRIPTION OF THE DRAWINGS

The above and other objects and features of the present invention will become apparent from the following description of the invention, when taken in conjunction with the accompanying drawings, which respectively show:
FIG. 1 shows a schematic diagram showing a process for preparing the drug-eluting stent of the present invention;
FIG. 2a shows an optical micrograph of a portion of the drug-eluting stent prepared in Example 1;
FIG. 2b shows the cross section of a drug-eluting stent and a scanning electron microscope photograph of surface of the drug-eluting stent prepared in Example 1; and
FIG. 3a to 3c show scanning electron microscope photographs of a biodegradable polymer surface before plasma treatment, a biodegradable polymer surface after plasma treatment at a bias voltage of -800 V and a drug-loaded biodegradable polymer surface after plasma treatment according to the present invention, respectively.

### DETAILED DESCRIPTION OF THE INVENTION

The inventive method for preparing a drug-eluting stent comprises: (a) forming a first biodegradable polymer layer on the surface of a stent; (b) forming a nanostructured pattern on the surface of the first biodegradable polymer layer by treatment with ion beams or plasma using a plasma-assisted chemical vapor deposition (PACVD); and, (c) forming a second biodegradable polymer layer on the first biodegradable polymer layer having the nanostructured pattern formed thereon, at least one of the first and second biodegradable polymer layers being loaded with identical or different drugs, wherein the ion beam or plasma treatment in step (b) is carried out at a voltage ranging from -100 V to -100 kV, wherein the ion beam or plasma treatment in step (b) is carried out at a power ranging from 1 W to 10 kW, and wherein the ion beam or plasma treatment in step (b) is carried out for a time ranging from 1 second to 2 hours. In the inventive method, the first biodegradable polymer layer may be loaded with a drug by loading a drug into the first biodegradable polymer layer having the nanostructured pattern formed thereon, obtained from step (b). Also, the first biodegradable polymer layer may be loaded with a drug by coating a drug-loaded biodegradable polymer on the surface of the stent, in step (a). Herein, the first biodegradable polymer layer may be further loaded with a drug by loading a second drug into the first biodegradable polymer layer having the nanostructured pattern formed thereon, obtained from step (b).

In the inventive method, the second biodegradable polymer layer may be loaded with a drug by coating a drug-loaded biodegradable polymer on the first biodegradable polymer layer having the nanostructured pattern formed thereon, in step (c). Herein, the first biodegradable polymer layer may also be loaded with at least one drug in the same manner as described in the preceding paragraph.

The inventive method for preparing the drug-eluting stent will now be described in further detail.

In step (a) or (c), the first and second biodegradable polymer layers can be formed by coating on the stent surface to a thickness of 10-20 µm using a spraying method (Chen et al., J of controlled release, 108:178-189, 2005). Herein, the biodegradable polymer may be a drug-loaded biodegradable polymer.

The stent may be made of a material which is conventionally used as a material for stents, such as stainless steel or nitinol (NiTi), and the thickness thereof may vary as occasion demands.

The biodegradable polymer may be a polymer having excellent biodegradability and biocompatibility and is preferably selected from the group consisting of polyglycolic acid (PGA), poly-L-lactic acid (PLLA), poly-DL-lactic acid (PDLLA), poly(lactic acid-co-glycolic acid) (PLGA), poly-ε-caprolactone (PCL), polyamino acid, polyanhydride, polyorthoester, and copolymers thereof.

In step (b), the nanostructured surface is formed on the surface of the biodegradable polymer, coated on the surface of the stent in step (a), by treatment with ion beams or plasma using a plasma-assisted chemical vapor deposition (PACVD).

The ion beam or plasma treatment can be carried out using a material selected from the group consisting of argon (Ar), nitrogen (N₂), oxygen (O₂), tetrafluoromethane (CF₄), and mixtures thereof. The ion beam or plasma treatment is carried out at a voltage ranging from -100 V to -100 kV, preferably from -500 V to -1000 V, at a power ranging from 1 W to 10 kW, preferably from 100 W to 500 W, for a time ranging from 1 second to 2 hours, preferably 1 minute to 10 minutes.

The first biodegradable polymer layer subjected to the above-described ion beam or plasma treatment has a nanostructured pattern formed thereon, and the pattern may be nano-hole, nano-wrinkle, nano-hair or nano-network. The nanostructured pattern can have a width ranging from 200 nm to 1 µm, preferably 200 nm, and a height ranging from 100 nm to 500 nm, preferably 200 nm. The width and height of the nanostructured pattern on the surface may vary depending on various conditions.

The nanostructured pattern formed in step (b) can increase the bonding strength between the biodegradable polymer and the metal stent and improve drug-loading capability and drug elution rate.

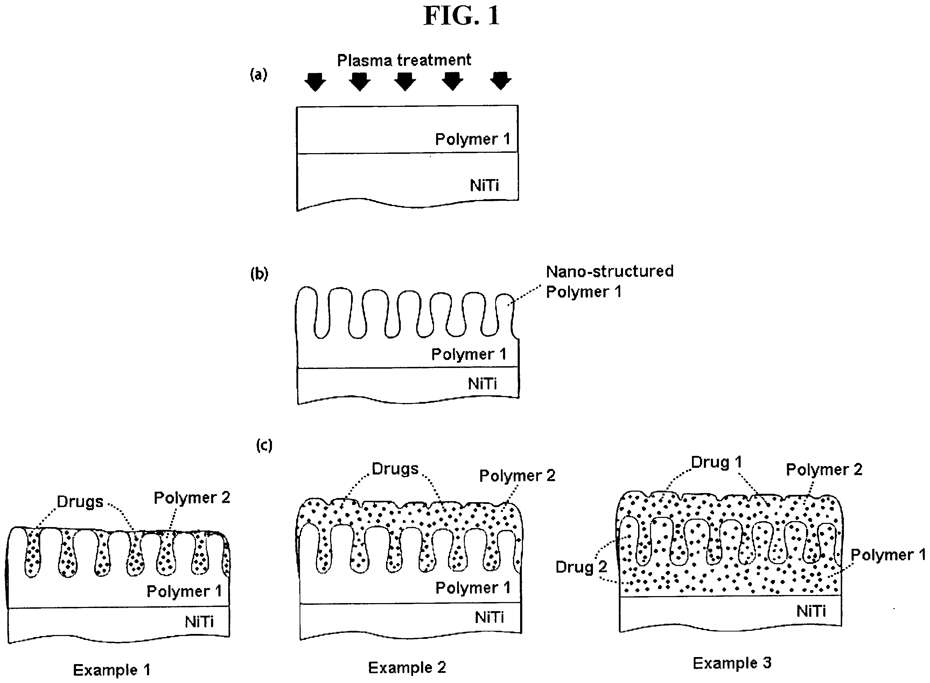

FIG. 1 is a schematic diagram showing a process of preparing a stent according to the embodiments of the present invention.

### EXAMPLES

Hereinafter, the present invention will be described in further detail with reference to examples. It is to be understood, however, that these examples are for illustrative purposes only and the scope of the present invention is not limited only to these examples.

### Example 1

### 1-1. Coating with biodegradable polymer

Poly(lactic acid-co-glycolic acid) (PLGA; Boehringer Ingelheim AG, Germany) (biodegradable polymer 1) was dissolved in methylene chloride (CH₂Cl₂) at a concentration of 10 wt%, and then coated on the surface of a laser-processed stent (Taewoong Medical Co., Ltd., Korea) to a thickness of 10 µm by a conventional spraying method (Chen et al., J of controlled release, 108:178-189, 2005).

### 1-2. Treatment with ion beams or plasma

The surface of the PLGA-coated stent, obtained in Example 1-1, was treated with argon plasma at a radio frequency of 13.56 MHz using a plasma-assisted chemical vapor deposition (PACVD). Specifically, the surface of the PLGA polymer layer was treated with argon (Ar) plasma at a chamber pressure of 1.33 Pa at a voltage of -800 V for 5 minutes, thus forming a nano-wrinkle structure. Herein, the structure may somewhat change depending on the treatment time and the chamber pressure.

### 1-3. Coating with drug-loaded biodegradable polymer

PLGA (Boehringer Ingelheim AG, Germany) (biodegradable polymer 2) was dissolved in methylene chloride at a concentration of 3 wt%, and then paclitaxel (Aldrich, 50 mg) (drug 1) was added and mixed therewith in an amount corresponding to 1/10 of PLGA. The mixed solution was coated on the surface of the biodegradable polymer layer having the nanostructured pattern formed thereon, obtained in Example 1-2, to a thickness of 2 µm using the same spraying method as in Example 1-1.

### Example 2

This Example was carried out in the same manner as in Example 1, except that, in Example 1-3, the biodegradable polymer 2 was coated to a thickness of 10 µm on the surface of the biodegradable polymer 1 having the nanostructured pattern formed thereon.

### Example 3

This Example was carried out in the same manner as in Example 1, except that, in the biodegradable polymer-coating step of Example 1-1, PLGA (Boehringer Ingelheim AG, Germany) (biodegradable polymer 1) was dissolved in toluene at a concentration of 10 wt%, and then paclitaxel (Aldrich, 50 mg) (drug 2) was added thereto, thereby coating on the surface of a laser-processed stent (Taewoong Medical Co., Ltd., Korea) to a thickness of 10 µm by a conventional spraying method.

### Experimental Example 1: Analysis of surface

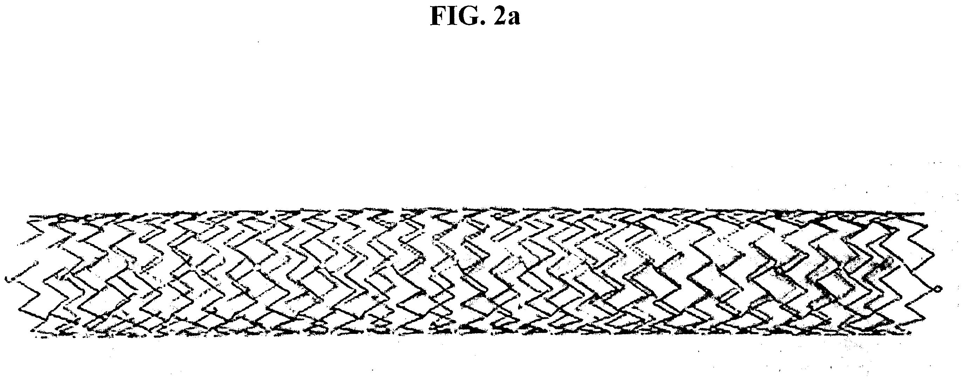

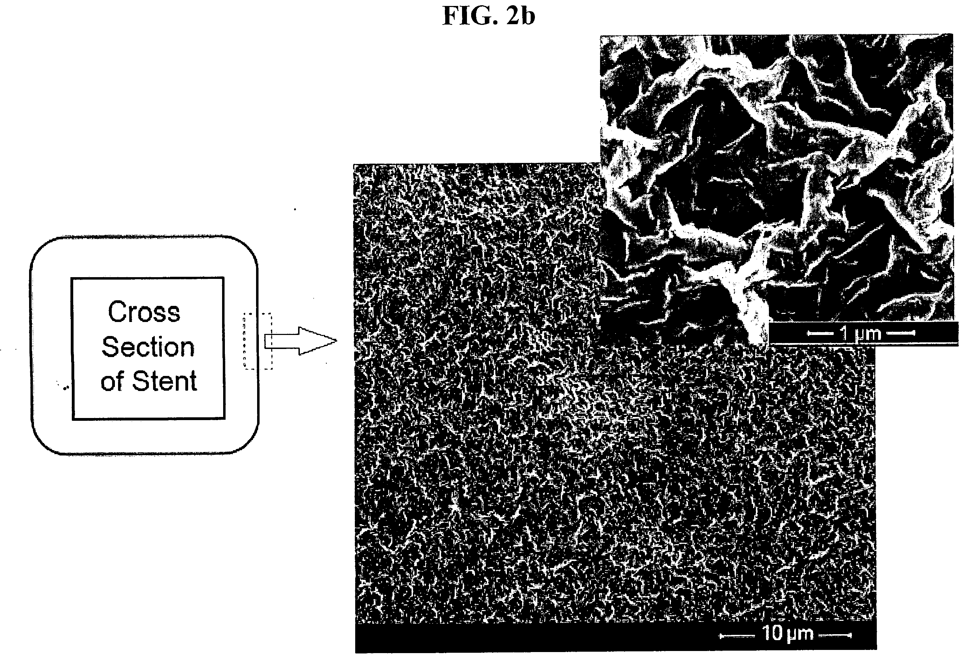

To analyze the surface of the stent obtained in Example 1, an atomic force microscope (AFM) (AutoProbe CP Research System, Thermo Microscope Inc., USA) was used, and the surface roughness of a 2 µm x 2 µm region was measured in a noncontact mode. The surface roughness was measured as a Root-Mean-Square (RMS). Also, the morphology of the surface was photographed with a scanning electron microscope (nano-SEM, FEI Inc.), and the results are shown in FIGS. 2a and 2b. FIG. 2a shows an optical microscopic image of a portion of the NiTi stent, and FIG. 2b shows the cross section of the stent and a scanning electron microscope photograph of surface thereof. As can be seen in FIG. 2b, a nanostructured pattern was formed on the surface of the stent. The surface nanostructured pattern had a width of about 200 nm and a height of about 100 nm. The width and height of the surface nanostructured pattern may vary depending on various conditions.

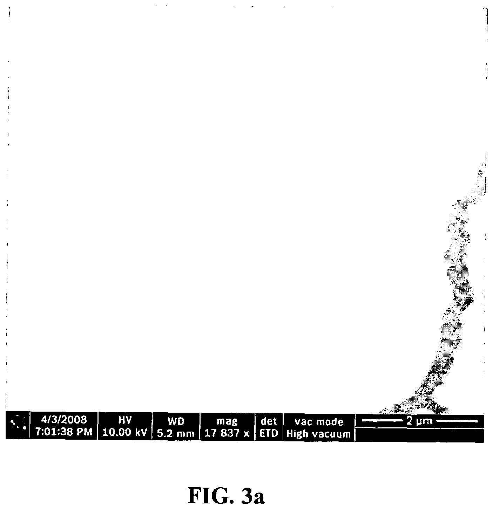

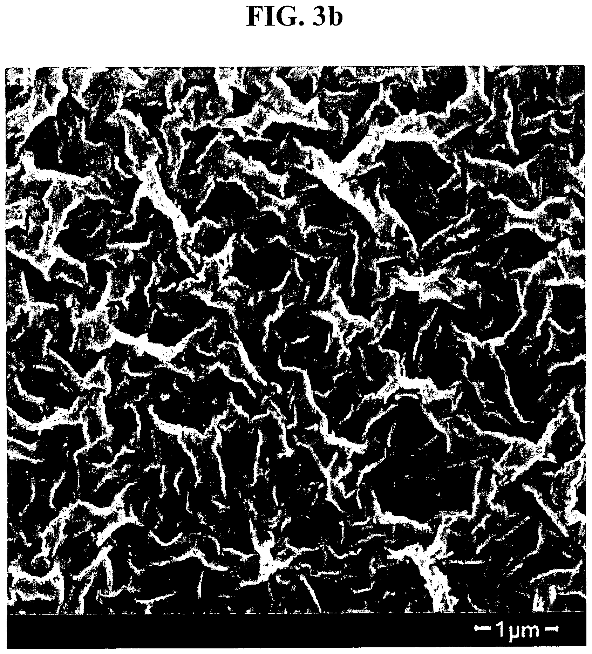

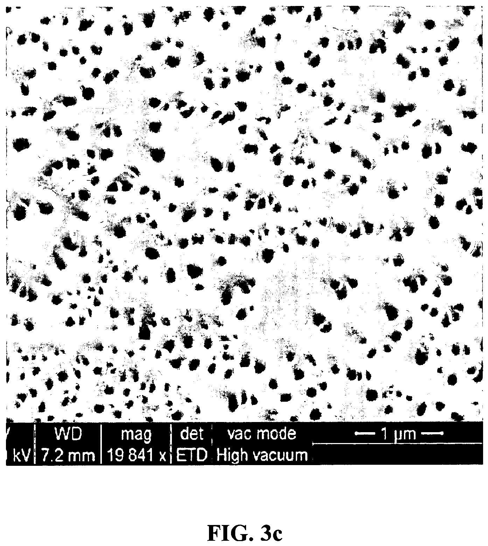

Also, a biodegradable polymer surface before plasma treatment, a biodegradable polymer surface after plasma treatment at a bias voltage of -800 V and a drug-loaded biodegradable polymer surface after plasma treatment according to the present invention were photographed with a scanning electron microscope (nano-SEM, FEI Inc.), and the results are shown in FIG. 3a to 3c. As can be seen in FIG. 3a to 3c, as compared to the smooth surface before plasma treatment (FIG. 3a), nano-sized patterns (FIG. 3b) and nano-sized holes (FIG. 3c) were formed by the plasma treatment.

## Claims

1. A method for preparing a drug-eluting stent, comprising the steps of:
(a) forming a first biodegradable polymer layer on the surface of a stent;
(b) forming a nanostructured pattern on the surface of the first biodegradable polymer layer by treatment with ion beams or plasma using a plasma-assisted chemical vapor deposition (PACVD); and,
(c) forming a second biodegradable polymer layer on the first biodegradable polymer layer having the nanostructured pattern formed thereon,
at least one of the first and second biodegradable polymer layers being loaded with identical or different drugs,
wherein the ion beam or plasma treatment in step (b) is carried out at a voltage ranging from -100 V to -100 kV,
wherein the ion beam or plasma treatment in step (b) is carried out at a power ranging from 1 W to 10 kW, and
wherein the ion beam or plasma treatment in step (b) is carried out for a time ranging from 1 second to 2 hours.

2. The method of Claim 1, wherein the first biodegradable polymer layer is loaded with a drug by loading a drug into the first biodegradable polymer layer having the nanostructured pattern formed thereon, obtained from step (b).

3. The method of Claim 1, wherein the first biodegradable polymer layer is loaded with a drug by coating a drug-loaded biodegradable polymer on the surface of the stent, in step (a).

4. The method of Claim 3, wherein the first biodegradable polymer layer is further loaded with a drug by loading a second drug into the first biodegradable polymer layer having the nanostructured pattern formed thereon, obtained from step (b).

5. The method of Claim 1, wherein the second biodegradable polymer layer is loaded with a drug by coating a drug-loaded biodegradable polymer on the surface of the first biodegradable polymer layer, in step (c).

6. The method of Claim 5, wherein the first biodegradable polymer layer is loaded with a drug by loading a drug into the first biodegradable polymer layer having the nanostructured pattern formed thereon, obtained from step (b).

7. The method of Claim 5, wherein the first biodegradable polymer layer is loaded with a drug by coating a drug-loaded biodegradable polymer on the surface of the stent, in step (a).

8. The method of Claim 7, wherein the first biodegradable polymer layer is further loaded with a drug by loading a second drug into the first biodegradable polymer layer having the nanostructured pattern formed thereon, obtained from step (b).

9. The method of Claim 1, wherein the first and second biodegradable polymer layers are formed by coating with a biodegradable polymer selected from the group consisting of polyglycolic acid (PGA), poly-L-lactic acid (PLLA), poly- DL-lactic acid (PDLLA), poly(lactic acid-co-glycolic acid) (PLGA), poly-e- caprolactone (PCL), polyamino acid, polyanhydride, polyorthoester, and copolymers thereof.

10. The method of Claim 1, wherein the ion beam or plasma treatment in step (b) is carried out using a material selected from the group consisting of argon (Ar), nitrogen (N₂), oxygen (O2), tetrafluoromethane (CF₄), and mixtures thereof.

11. The method of Claim 1, wherein the nanostructured pattern in step (b) is selected from the group consisting of nano-hole, nano-wrinkle, nano-hair and nano-network.

## Patentansprüche

1. Verfahren zur Herstellung eines Medikamente freisetzenden Stents, umfassend die folgenden Schritte:
(a) Ausbilden einer ersten biologisch abbaubaren Polymerschicht auf der Oberfläche eines Stents;
(b) Ausbilden eines nanostrukturierten Musters auf der Oberfläche der ersten biologisch abbaubaren Polymerschicht durch Behandlung mit Ionenstrahlen oder Plasma mittels plasmaunterstützter chemischer Gasphasenabscheidung (PACVD); und
(c) Ausbilden einer zweiten biologisch abbaubaren Polymerschicht auf der das darauf ausgebildete nanostrukturierte Muster aufweisenden ersten biologisch abbaubaren Polymerschicht,
wobei mindestens eine der beiden biologisch abbaubaren Polymerschichten mit identischen oder unterschiedlichen Medikamenten beladen wird,
wobei die Ionenstrahl- oder Plasmabehandlung in Schritt (b) bei einer Spannung von -100 V bis -100 kV erfolgt,
wobei die Ionenstrahl- oder Plasmabehandlung in Schritt (b) bei einer Leistung von 1 W bis 10 kW erfolgt und
wobei die Ionenstrahl- oder Plasmabehandlung in Schritt (b) über einen Zeitraum von 1 Sekunde bis 2 Stunden erfolgt.

2. Verfahren nach Anspruch 1, wobei die Beladung der ersten biologisch abbaubaren Polymerschicht mit einem Medikament durch Laden eines Medikaments in die erste biologisch abbaubare Polymerschicht mit dem darauf ausgebildeten nanostrukturierten Muster, welche in Schritt (b) erhalten wurde, erfolgt.

3. Verfahren nach Anspruch 1, wobei die Beladung der ersten biologisch abbaubaren Polymerschicht mit einem Medikament durch Beschichtung der Oberfläche des Stents mit einem mit einem Medikament beladenen biologisch abbaubaren Polymer in Schritt (a) erfolgt.

4. Verfahren nach Anspruch 3, wobei eine weitere Beladung der ersten biologisch abbaubaren Polymerschicht mit einem Medikament durch Laden eines zweiten Medikaments in die erste biologisch abbaubare Polymerschicht mit dem darauf ausgebildeten nanostrukturierten Muster, welche in Schritt (b) erhalten wurde, erfolgt.

5. Verfahren nach Anspruch 1, wobei die Beladung der zweiten biologisch abbaubaren Polymerschicht mit einem Medikament durch Beschichtung der Oberfläche der ersten biologisch abbaubaren Polymerschicht mit einem mit einem Medikament beladenen biologisch abbaubaren Polymer in Schritt (c) erfolgt.

6. Verfahren nach Anspruch 5, wobei die Beladung der ersten biologisch abbaubaren Polymerschicht mit einem Medikament durch Laden eines Medikaments in die erste biologisch abbaubare Polymerschicht mit dem darauf ausgebildeten nanostrukturierten Muster, welche in Schritt (b) erhalten wurde, erfolgt.

7. Verfahren nach Anspruch 5, wobei die Beladung der ersten biologisch abbaubaren Polymerschicht mit einem Medikament durch Beschichtung der Oberfläche des Stents mit einem mit einem Medikament beladenen biologisch abbaubaren Polymer in Schritt (a) erfolgt.

8. Verfahren nach Anspruch 7, wobei eine weitere Beladung der ersten biologisch abbaubaren Polymerschicht mit einem Medikament durch Laden eines zweiten Medikaments in die erste biologisch abbaubare Polymerschicht mit dem darauf ausgebildeten nanostrukturierten Muster, welche in Schritt (b) erhalten wurde, erfolgt.

9. Verfahren nach Anspruch 1, wobei die erste und die zweite biologisch abbaubare Polymerschicht durch Beschichtung mit einem biologisch abbaubaren Polymer ausgebildet werden, welches aus der Gruppe bestehend aus Polyglycolsäure (PGA), Poly-L-Milchsäure (PLLA), Poly-D,L-Milchsäure (PDLLA), Poly(lactid-co-glycolid) (PLGA), Poly-ε-Caprolacton (PCL), Polyaminosäure, Polyanhydrid, Polyorthoester und deren Copolymeren ausgewählt ist.

10. Verfahren nach Anspruch 1, wobei die Ionenstrahl- oder Plasmabehandlung in Schritt (b) mittels eines Materials durchgeführt wird, das aus der Gruppe bestehend aus Argon (Ar), Stickstoff (N₂), Sauerstoff (O₂), Tetrafluormethan (CF₄) und Mischungen derselben ausgewählt ist.

11. Verfahren nach Anspruch 1, wobei das nanostrukturierte Muster in Schritt (b) aus der Gruppe bestehend aus Nanolöchern, Nanofalten, Nanohaaren und Nanonetzen ausgewählt ist.

## Revendications

1. Procédé de préparation d'un stent à élution de médicaments, comprenant les étapes suivantes :
(a) former une première couche polymère biodégradable sur la surface d'un stent ;
(b) former un motif nanostructuré sur la surface de la première couche polymère biodégradable à l'aide d'un traitement par faisceaux d'ions ou plasma en utilisant un dépôt chimique en phase vapeur assisté par plasma (PACVD) ; et
(c) former une deuxième couche polymère biodégradable sur la première couche polymère biodégradable sur laquelle est formé le motif nanostructuré,
au moins une des première et deuxième couches polymères biodégradables étant chargée de médicaments identiques ou différents,
dans lequel le traitement par faisceau d'ions ou plasma dans l'étape (b) est effectué sous une tension allant de -100 V à -100 kV,
dans lequel le traitement par faisceau d'ions ou plasma dans l'étape (b) est effectué à une puissance allant de 1 W à 10 kW, et
dans lequel le traitement par faisceau d'ions ou plasma dans l'étape (b) est effectué pendant un temps allant de 1 seconde à 2 heures.

2. Procédé selon la revendication 1, dans lequel la première couche polymère biodégradable est chargée d'un médicament en chargeant un médicament dans la première couche polymère biodégradable sur laquelle est formé le motif nanostructuré, obtenue de l'étape (b).

3. Procédé selon la revendication 1, dans lequel la première couche polymère biodégradable est chargée d'un médicament en revêtant la surface du stent d'un polymère biodégradable chargé d'un médicament, dans l'étape (a).

4. Procédé selon la revendication 3, dans lequel la première couche polymère biodégradable est chargée d'un médicament de manière supplémentaire en chargeant un deuxième médicament dans la première couche polymère biodégradable sur laquelle le motif nanostructuré est formé, obtenue de l'étape (b).

5. Procédé selon la revendication 1, dans lequel la deuxième couche polymère biodégradable est chargée d'un médicament en revêtant la surface de la première couche polymère biodégradable d'un polymère biodégradable chargé d'un médicament, dans l'étape (c).

6. Procédé selon la revendication 5, dans lequel la première couche polymère biodégradable est chargée d'un médicament en chargeant un médicament dans la première couche polymère biodégradable sur laquelle est formé le motif nanostructuré, obtenue de l'étape (b).

7. Procédé selon la revendication 5, dans lequel la première couche polymère biodégradable est chargée d'un médicament en revêtant la surface du stent d'un polymère biodégradable chargé d'un médicament, dans l'étape (a).

8. Procédé selon la revendication 7, dans lequel la première couche polymère biodégradable est chargée d'un médicament de manière supplémentaire en chargeant un deuxième médicament dans la première couche polymère biodégradable sur laquelle le motif nanostructuré est formé, obtenue de l'étape (b).

9. Procédé selon la revendication 1, dans lequel les première et deuxième couches polymères biodégradables sont formées en étant revêtues d'un polymère biodégradable sélectionné dans le groupe consistant à l'acide polyglycolique (PGA), le poly-L-acide lactique (PLLA), le poly-D,L-acide lactique (PDLLA), l'acide poly(lactique-co-glycolique) (PLGA), la poly-ε-caprolactone (PCL), le polyaminoaacide, le polyanhydride, le polyorthoester, et des copolymères de ceux-ci.

10. Procédé selon la revendication 1, dans lequel le traitement par faisceau d'ions ou plasma dans l'étape (b) est effectué en utilisant un matériau sélectionné dans le groupe consistant à l'argon (Ar), l'azote (N₂), l'oxygène (O₂), le tétrafluorure de carbone (CF₄), et des mélanges de ceux-ci.

11. Procédé selon la revendication 1, dans lequel le motif nanostructuré dans l'étape (b) est sélectionné dans le groupe consistant à des nano-trous, des nano-plis, des nano-cheveux et des nanoréseaux.
